(19) **Europäisches Patentamt / European Patent Office / Office européen des brevets**

(11) **EP 2 320 842 B1**

(12) # EUROPEAN PATENT SPECIFICATION

(45) Date of publication and mention of the grant of the patent:
**27.06.2012 Bulletin 2012/26**

(51) Int Cl.:
*A61F 9/007* (2006.01)   *A61M 1/00* (2006.01)
*A61B 17/32* (2006.01)   *A61B 3/16* (2006.01)

(21) Application number: **09762190.8**

(22) Date of filing: **08.06.2009**

(86) International application number:
**PCT/IT2009/000248**

(87) International publication number:
**WO 2009/150683 (17.12.2009 Gazette 2009/51)**

(54) **DEVICE FOR OPHTHALMIC SURGERY, IN PARTICULAR FOR CATARACT REMOVAL, PROVIDED WITH A SYSTEM FOR DYNAMICALLY MAINTAINING INTRAOCULAR PRESSURE**

VORRICHTUNG FÜR DIE OPHTHALMISCHE CHIRURGIE, INSBESONDERE ZUR KATARAKTENTFERNUNG MIT EINEM SYSTEM ZUR DYNAMISCHEN AUFRECHTERHALTUNG DES AUGENINNENDRUCKS

DISPOSITIF DE CHIRURGIE OPHTALMIQUE, DESTINÉ EN PARTICULIER À UNE SUPPRESSION DE CATARACTE, COMPORTANT UN SYSTÈME DE MAINTIEN DYNAMIQUE DE LA PRESSION INTRAOCULAIRE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **10.06.2008 IT RM20080300**

(43) Date of publication of application:
**18.05.2011 Bulletin 2011/20**

(73) Proprietor: **Optikon 2000 S.p.A.**
**00138 Roma (IT)**

(72) Inventors:
• **ANGELINI, Giovan, Battista**
**I-00138 Roma (IT)**

• **CAMERLENGO, Flavio**
**I-00138 Roma (IT)**
• **PUGLIESE, Giuseppe**
**I-00138 Roma (IT)**

(74) Representative: **Santi, Filippo et al**
**Barzanò & Zanardo Roma S.p.A.**
**Via Piemonte, 26**
**IT-00187 Roma (IT)**

(56) References cited:
**US-A- 4 340 037      US-A- 5 722 945**
**US-A- 5 830 176      US-A1- 2002 099 400**
**US-B1- 6 491 661**

## Description

The present invention relates to a device for ophthalmic surgery, in particular for cataract removal through a corneal incision of small diameter.

More in particular, the invention relates to a handpiece or handle to use in cataract removal technique by using high vacuum and low mechanical energy, provided with a dynamic system for maintaining intraocular pressure IOP.

Presently, the most spread surgical technique for cataract removal is "phacoemulsification". A "phacoemulsificator" is substantially a tool composed of a control unit and a "handpiece" or "handle" through which a surgeon performs an intervention.

A "needle" or "tip" is connected at the handpiece end, which tip is put in oscillation with ultrasound frequency by a "piezoelectric" or "magnetostrictive" system housed within the same "handpiece".

The handpiece," is also provided with irrigation and suction systems, the first one of which operates by gravity, whereas the second one is assisted by a vacuum pump, generally a "Venturi"-type or peristaltic one.

The suction system i s' made in such a manner to coaxially operate through the needle and it serves for aspirating the crystalline (cataract) fragmented by the vibrational motion of the needle.

Instead, the irrigation system operates concentrically and externally to the needle irrigating the eye with a physiological solution for restoring the liquid aspirated along with the crystalline.

In particular, the irrigation passes through the annular space created by a small "sleeve", generally in silicone, coaxially arranged externally to the needle.

Present trend in cataract "phacoemulsification" is to reduce as much as possible the ultrasound emission energy of the tip and to simultaneously work with high vacuum.

The use of high vacuum, especially with "Venturi"-type suction systems, in which it is not possible to control flow independently from the set vacuum, creates instability problems within the eye.

In a "traditional" phacoemulsification system under conditions of not occluded tip, the vacuum level present within the suction line is not very high (40-60 mmHg, where 1 mmHg = 133,322 Pa). Such vacuum applied to the suction line generates a suction flow that is highly balanced by the irrigation one provided by gravity from the bottle of saline solution.

When the tip captures a crystalline fragment and gets occluded, flow stops and vacuum within the suction line rises up to the value set by the surgeon.

When the occlusion dissolves, a suction flow is rapidly generated that is directly proportional to the reached vacuum level and inversely proportional to the impedance of the suction line.

Elasticity of the suction line negatively takes part in this, since it release the energy stored during occlusion in the form of (negative) pressure when the occlusion dissolves.

If the vacuum selected by the surgeon is high and in fact this is the present surgical trend, the flow generated by gravity from the irrigation bottle could not be able to balance the suction one and a collapse within the eye occurs.

Since the impedance of the suction line is most located in the tip it is evident that the several proposed solutions focus on tip geometry.

One of the proposed solutions is to make a diaphragm within the tip in positions more or less backwards with respect to the end of the same tip; patent applications US2001/0031951A1 by Pezzola, US2002/0099325A1 by Sutton and also the several geometries illustrated in patent US 6,299,591 B1 to Banko follow this route.

On the contrary, all these geometries create problems of tip occlusion since the fragment of crystalline "captured" by the tip is forced to pass through a neck within the tip.

All the above goes against the present trend of reducing to a minimum the use of ultrasounds, since the surgeon must frequently apply ultrasounds (oscillation) to the tip for removing the occlusion.

Another route followed in order to reduce pressure instability within the eye under operation and to avoid tip occlusions has been to increase the impedance on the suction line after the handpiece, by reducing the pipe bore and creating chambers for collecting the removed tissue for avoiding the occlusion of the suction line (Baush & Lomb).

Patent US 5167620 to Ureche also belongs to this tendency, disclosing a system having a suction line with variable impedance as a function of the pressure variations within the same line.

Others disclose solutions provided with flow "dampers" still on the suction line, as in patent US 4921477 to Davis.

As far as the solutions relating to irrigation line, generally fed by "gravity", are concerned, besides the common tendency of decreasing the hydraulic impedance of the same line, the one proposed in patent US 6042586 to Kawano is particularly interesting, which exploits the energy of elastic deformation of a latex chamber, arranged on the irrigation line in proximity to the handpiece.

Other solutions provide the use of pressures "added" to the "hydrostatic" one, as in Italian patent application No. RM2006A000477 by Optikon providing an irrigation pocket held under pressure by blown air.

The solution illustrated in patent US6491661B1 to Bouchny provides the use of a flow sensor placed in the irrigation line which substantially checks whether the flow remains within the set values and, should it vary, besides emitting an alarm, it modifies the values of both suction and irrigation flow for maintaining the initial value; however, the limit of such solution is that it exclusively controls the irrigation flow and not the IOP, that nevertheless can undergo variations under operation.

With the aim of completely eliminating the use of oscillations with ultrasound frequency which moreover transmit a harmful energy to ocular tissues, many alternative techniques have been developed for cataract removal: techniques providing the use of conveyed water jets "Aqualase", as disclosed in patent application No. WO9825557 by Alcon; techniques providing the use of laser beams, as disclosed in patent applications US2006084961, WO9812973, DE19718139, CA2113911 and W08906522 and in patent US5843071; techniques providing mechanical actions of various nature on the cataract, such actions as those combined with water jets, as disclosed in patent US7041078 and in patent application US2005251105 by PEYMAN GHOLAM, dilatation of micro balloons, as disclosed in patent US5695461 to SCHAIBLE ERIC, "strip" circular blades, as disclosed in patent application DE4012882, or through suction rotary pumps, as disclosed in patent application DE1971167A1.

Document US 5830176 discloses a device for controlling intraocular pressure that is based on a switching valve system which selects one or more sources out of a plurality of external sources at different pressures.

Document US 5722945 discloses a device for removing "pathological tissues" without damaging healthy tissues, wherein discrimination is based on the different mechanical response of tissues to the cutting action. Such device is provided with blades not having circular symmetry and operated so as to perform a rotary motion in one direction only at ultrasound frequency.

In this context, the solution proposed according to the present invention is introduced.

Hence, it is an object of the present invention to allow, in a simple and reliable way, to maintain an intraocular pressure IOP as constant as possible during cataract removal.

It is therefore specific subject matter of this invention a device for ophthalmic surgery, in particular for cataract removal, with intraocular pressure (IOP) stabilisation, as defined in independent claim 1.

Further embodiments of the device for ophthalmic surgery are defined in the dependent claims 2-15.

Still according to the invention, the device may be capable to aspirate a cataract and the tip may be operated so as to perform a reciprocating rotary motion capable to generate, due to inertial effect, a rotation of a cataract with respect to the tip.

In particular, said reciprocating rotary motion may be also used in a device for ophthalmic surgery, in particular for cataract removal, independently from intraocular pressure (IOP) stabilisation and specific shape of the handpiece and related tip.

The device does not transmit harmful energy forms to the eye and it is capable to operate with high suction values maintaining constant intraocular pressure (IOP).

Preferably, the device is a handpiece provided with a particular tip already used for cataract phacoemulsification, suitably modified. The tip under consideration, subject matter of the European patent EP1212995 by Optikon characterised by a circular symmetry, cutting profile and "anti-occlusion" geometry, is modified for optimising it to the particular "motion" with which it is used in the present invention.

The present invention will be now described, by way of illustration and not by way of limitation, according to its preferred embodiments, by particularly referring to the Figures of the enclosed drawings, in which:

Figure 1 shows a part sectional perspective view (Fig. 1 a) and a front view (Fig. 1 b) of the tip employed in a first embodiment of the device according to the invention;
Figure 2 shows a part sectional perspective view of the tip of Figure 1 interacting with a cataract fragment;
Figure 3 shows a schematic block diagram of a first embodiment of the device according to the invention;
Figure 4 shows a schematic block diagram of a second embodiment of the device according to the invention;
Figure 5 shows a part sectional perspective view of the device of Figure 3; and
Figure 6 shows a part sectional perspective view of the device of Figure 4.

In the Figures, identical reference numbers are used for alike elements.

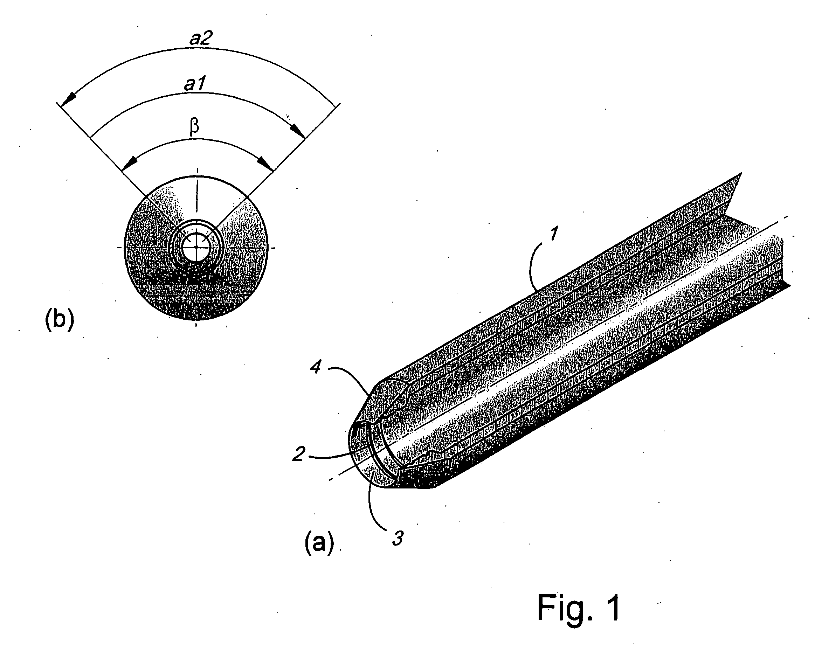

Figure 1 shows a part sectional perspective view (Fig. 1 a) and a front view (Fig. 1 b) of the tip 1 that is part of a first embodiment of the device, namely a handpiece, according to the invention. In the distal portion extending from the end of the tip 1 to the inside of the same, the tip 1 comprises a diaphragm 3 internally provided with a helicoidal scoring 2, preferably with pitch ranging from 0,1 to 0,4 mm, more preferably equal to 0,2 mm. A conicity 4 is made on the tip 1 outside the diaphragm 3, wherein the axial length of which conicity is not longer than, preferably equal to, the one of the diaphragm 3. This conicity 4 is aimed at creating a circular "cutting" edge that gives the end of the tip 1 an optimal capability of "indenting" the crystalline.

As schematically sho wn in Figure 1 b, the tip 1 is operated according to an asymmetrical reciprocating rotary motion that makes it describe an arc of a circle, subtending an angle $\beta$, with different angular accelerations, respectively equal to $a_1$ and $a_2$, in the two directions of rotation, such that, preferably:

$$a_1 < a_2$$

As a consequence, the time intervals, respec-

tively equal to $t_1$ and $t_2$, necessary to the tip 1 for covering the angle β in the two directions are different. In particular, such time intervals are tied to the accelerations by the relationship imposing the same covered angle β:

$$1/2 \cdot a_1 \cdot t_1{}^2 = 1/2 \cdot a_2 \cdot t_2{}^2$$

whereby

$$a_1/a_2 = t_2{}^2/t_1{}^2 \quad [1].$$

Hence, the tip 1 moves according to a reciprocating rotary motion with angular amplitude β, characterised by different accelerations in the two running directions and by running intervals inversely proportional to said accelerations according to the previous relationship [1].

The motion in question may be transmitted to the tip 1 through an electric motor or a system of different nature, e.g. a pneumatically operated one, a hydraulic one or other.

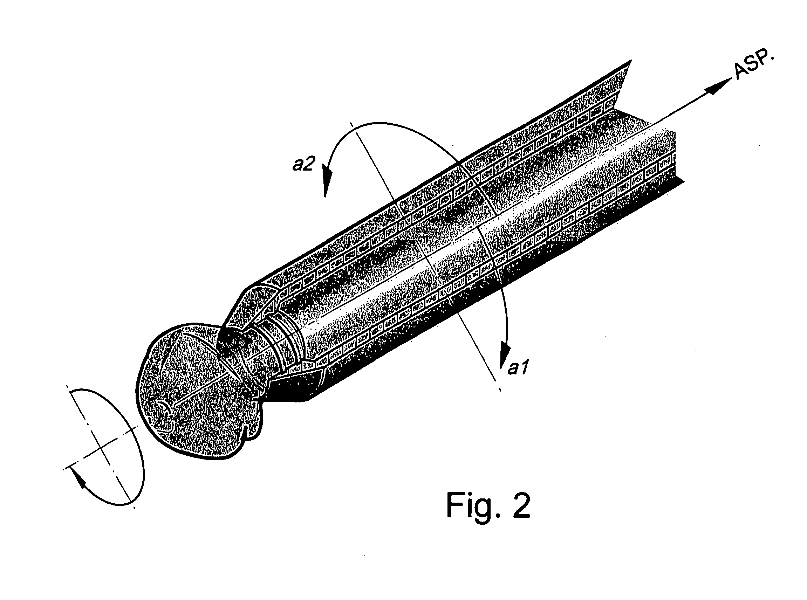

As shown in Figure 2, such asymmetrical reciprocating rotary motion of the tip 1 carries out a relative rotary motion between the tip 1 and the cataract particle with which the former interacts exploiting the inertia of the same particle. By properly combining with each other the three characteristic parameters of the motion, i.e. angular amplitude of the oscillation, frequency and accelerations, it is possible to obtain a relative rotary motion between the tip 1 and the cataract even with relatively small masses of the latter.

This relative rotary motion makes the tip 1 perform a double action: a cutting action, operated by the end of the same tip, and a dragging action, made by the helicoidal scoring 2 within the diaphragm 3. This double action, combined with the suction effect, performs the cataract removal avoiding persistent occlusions of the tip 1, without transmitting any appreciable energy to the eye tissues.

Especially in case of hard cataracts, it is necessary to operate with high vacuum values which may generate sudden variations of suction flow. In order to keep the intraocular pressure as constant as possible during operation in presence of rapid variations of the suction flow, the device according to the invention is provided with a system of "active" compensation of the intraocular pressure (IOP), that is integrated in the same handpiece.

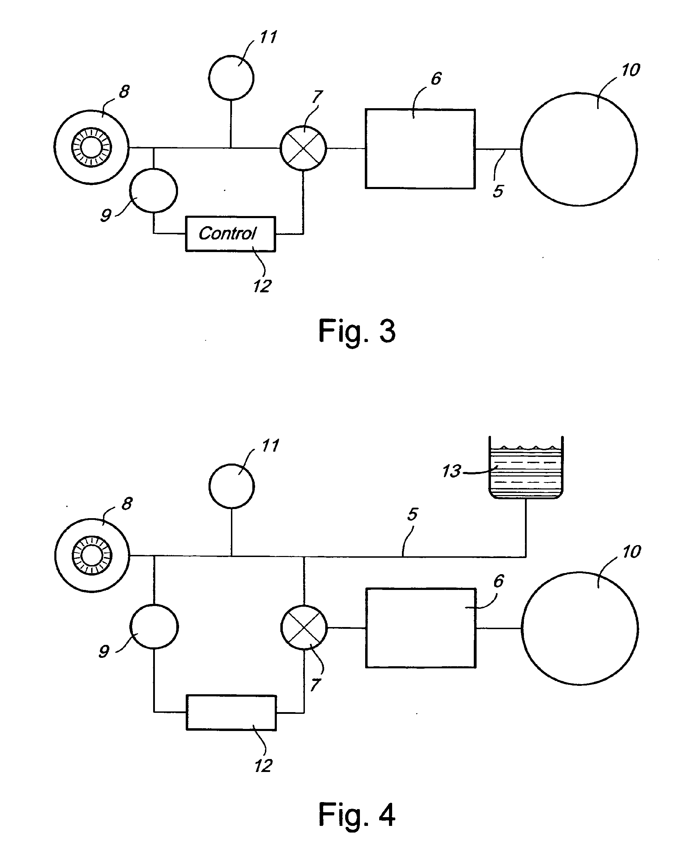

With reference to Figure 3, it may be observed a schematic block diagram of a first embodiment of the device according to the invention, where only the irrigation line is represented. Along the irrigation line 5, preferably within the handpiece, a chamber 6 is made, that is separated from the following hydraulic circuit through an intercepting valve 7 capable to put it in communication with the eye 8; in particular, the chamber 6 may have either rigid or elastic walls. A pressure sensor 9 is located after the valve 7, which sensor senses the intraocular pressure instant by instant.

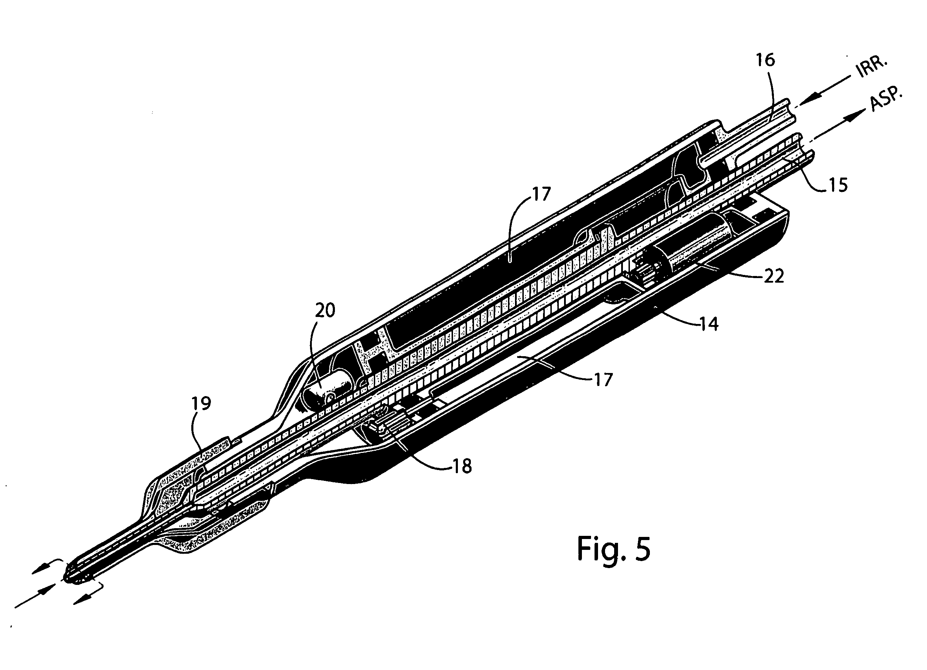

The irrigation fluid is maintained within the chamber 6 at a pressure sufficiently higher than the ocular one that is desired to maintain constant, in proportion to the volume of the same chamber 6, by a generator 10. Preferably, the pressure at which the generator 10 maintain the irrigation fluid within the chamber 6 is adjustable. The generator 10 may be constituted by either a "gravity" system, using the hydrostatic pressure of the irrigation fluid contained within a container placed at a certain height, or a pressure generator, i.e. a dynamic or volumetric hydraulic generator (hydraulic pump), located along the irrigation line. Use of a hydraulic condenser 11 after the valve 7 may also be provided; such condenser 11, preferably made as an elastic chamber, may be constituted by the same irrigation sleeve 19 shown in Figure 5, that shows a part sectional perspective view of the device of Figure 3.

Still making reference to Figure 3, it may be observed that the pressure sensor 9 continuously controls the intraocular pressure (IOP) and allows a control unit 12 to drive the intercepting valve 7 on the basis of the IOP sensed by the sensor 9, thus maintaining a constant pressure during operation. Preferably, the intercepting valve 7 is a solenoid valve controlled by a control electronics, implementing the control unit, that activates the solenoid valve depending on the pressure sensed by the sensor 9, so as to stabilise it at the desired IOP value. Preferably, the desired IOP value is adjustable.

With reference to Figure 4, a schematic block diagram of a second embodiment of the device according to the invention may be observed, wherein again only the irrigation line is represented. A pressure sensor 9 sensing the intraocular pressure instant by instant is placed along the irrigation line 5, preferably within the handpiece, and in proximity to the eye 8. The irrigation line 5 may be fed by a "gravity" system, using the hydrostatic pressure (preferably of adjustable value) of the irrigation fluid contained within a container 13 placed at a certain height.

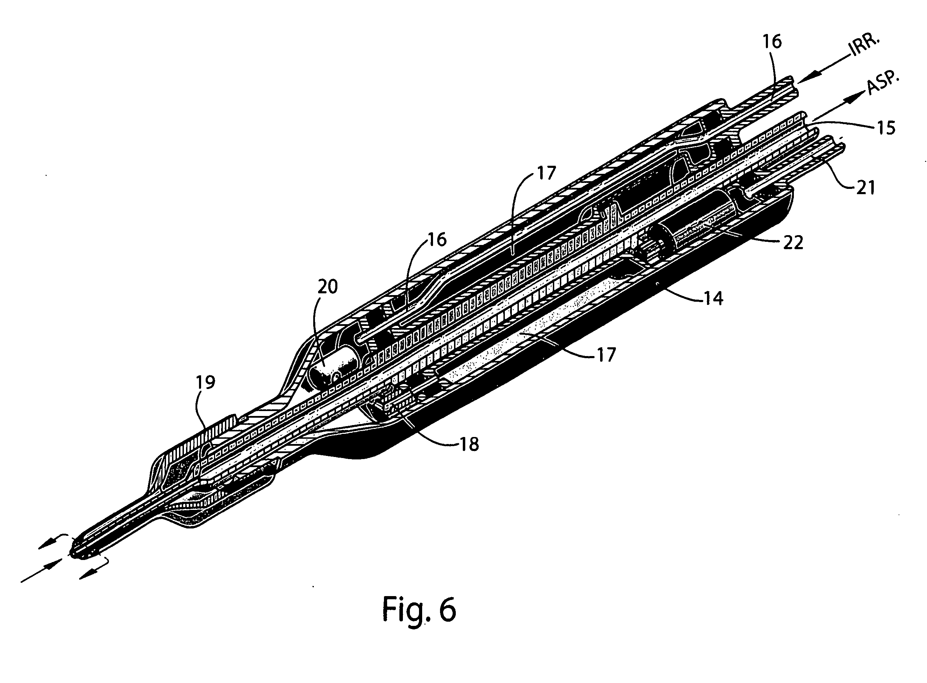

A chamber 6, separated from the irrigation line 5 through a valve 7 (preferably a solenoid valve), is placed in parallel to the line 5, preferably within the handpiece; in particular, the chamber 6 may have either rigid or elastic walls. The irrigation fluid is maintained within the chamber 6 at a pressure sufficiently higher than the ocular one that is desired to maintain constant, in proportion to the volume of the same chamber 6, by a generator 10. Preferably, the pressure at which the generator 10 maintain the irrigation fluid within the chamber 6 is adjustable. The generator 10 may be constituted by either a "gravity" system, using the hydrostatic pressure of the irrigation fluid contained within a corresponding container placed at a certain height, or a pressure generator, i.e. a dynamic or volumetric hydraulic generator (hydraulic pump), located before the chamber 6. Use of a hydraulic condenser 11 after the valve 7 may also be provided; such condenser, preferably made as an elastic chamber, may be constituted by the same irrigation sleeve 19 shown in Figure 6, that shows a part sectional perspective view of the Device of Figure 4.

Still making reference to Figure 4, the pressure sensor 9 continuously senses the intraocular pressure (IOP) and allows a control (preferably electronic) unit 12 to maintain a constant intraocular pressure, by opening, when the IOP decreases below a (preferably adjustable) determined value, the intercepting valve 7 thus causing an increase of pressure, and hence of flow, within the irrigation line 5.

The very fast response of the IOP compensation system and the reduced inertia of the irrigation line 5 after the valve 7 make the device according to the invention, particularly according to the two embodiments of Figures 3 and 4, very effective in stabilising IOP during cataract removal operation.

Figure 5 shows a part sectional perspective view of the device of Figure 3, provided with the IOP active compensation system. The device is composed of a handpiece 14 for cataract removal provided with a coaxial suction line 15 and an irrigation line 16. A chamber 17, separated from the tip portion by an intercepting valve 18 located after the same chamber 17, capable to put it in communication with the eye through the irrigation sleeve 19, is made along the irrigation line 16, within the handpiece 14. A pressure sensor 20 is located after the valve, sensing the intraocular pressure instant by instant.

The irrigation fluid within the chamber 17 is maintained at a pressure sufficiently higher than the ocular one that is desired to maintain constant, in proportion to the volume of the same chamber 17. The (preferably adjustable) pressure within the chamber 17 may be maintained by either a "gravity" system, using the hydrostatic pressure of the irrigation fluid contained within a container placed at a certain height, or a pressure generator, i.e. a dynamic or volumetric hydraulic generator (hydraulic pump), located along the irrigation line 16, externally to the handpiece 14.

The pressure sensor 20 continuously controls the intraocular pressure (IOP) and consequently drive the intercepting valve 18 maintaining a constant pressure during operation (equal to a preferably adjustable value).

The electric motor 22 operating the tip of the handpiece 14, making it perform the asymmetrical reciprocating rotary motion, is further shown in Figure 5.

La Figure 6 shows a part sectional perspective view of the device of Figure 4, wherein the handpiece 14 provided with a coaxial suction line 15 and an irrigation line 16 is illustrated. Line 16 is fed with the irrigation fluid contained in a container placed at a certain height, so as to balance the suction flow in dynamic conditions (without occlusions).

A chamber 17, separated from the irrigation line 16 through an intercepting valve 18 is made within the handpiece. The irrigation fluid within the chamber 17 is maintained at a (preferably adjustable) pressure sufficiently higher than the one of the irrigation line 16 by either a "gravity" system, exploiting the hydrostatic pressure of a container placed at a certain height, or a hydraulic generator connected to the inlet 21 of the handpiece 14. The pressure sensor 20 continuously controls the intraocular pressure (IOP), and when the latter decreases below a (preferably adjustable) determined value it opens the intercepting valve 18 putting the chamber 17 in communication with the irrigation line 16, causing an increase of pressure, and hence of flow, within the irrigation line 16, thus maintaining a constant intraocular pressure (equal to a preferably adjustable value).

The electric motor 22 operating the tip of the handpiece 14, making it perform the asymmetrical reciprocating rotary motion, is also further shown in Figure 6.

The very fast response of the IOP compensation system and the reduced inertia of the suction line 15 after the valve 18 make the system very effective in stabilising IOP during cataract removal operation.

The preferred embodiments have been above described and some modifications of this invention have been suggested, but it should be understood that those skilled in the art can make variations and changes, without so departing from the related scope of protection, as defined by the following claims.

## Claims

1. Device for ophthalmic surgery, in particular for cataract removal, with intraocular pressure (IOP) stabilisation, comprising a handpiece (14) internally including an irrigation line (5), within which an irrigation fluid flows, having an end portion capable to irrigate an eye, the handpiece additionally comprising one first source of irrigation fluid comprising a chamber (14) (6; 17) wherein the irrigation fluid is maintained at a first pressure value wherein the chamber (6: 17) is connected through valve means (7; 18) to said end portion of the irrigation line (5), said valve means (7; 18) being internally included in the handpiece and being controlled by control means (12) on the basis of a pressure detection provided by pressure sensor means (9; 20) connected to said end portion of the irrigation line (5) and capable to sense an intraocular pressure (IOP), whereby said control means (12) opens said valve means (7; 18) when the pressure sensed by said sensor means (9; 20) is not higher than a second pressure value not higher than the first pressure value, thus putting said chamber (6; 17) in communication with said end portion of the irrigation line (5).

2. Device according to claim 1, **characterised in that** said second pressure value is lower than said first pressure value.

**3.** Device according to claim 1 or 2, **characterised in that** one out of or both said first and second pressure values are adjustable.

**4.** Device according to any one of the preceding claims, **characterised in that** it further comprises a generator (10) capable to maintain said irrigation fluid within the chamber (6) at said first pressure value, wherein the generator (10) preferably comprises:

- a "gravity" system using a hydrostatic pressure of the irrigation fluid contained within a container placed at a certain height, and/or
- a dynamic or volumetric hydraulic generator, preferably a hydraulic pump.

**5.** Device according to any one of the preceding claims, **characterised in that** said valve means (7; 18) comprises a solenoid valve and **in that** said control means comprises an electronic unit (12) for controlling the solenoid valve.

**6.** Device according to any one of the preceding claims, **characterised in that** it further comprises hydraulic condenser means (11) connected to said end portion of the irrigation line (5) after said valve means (7; 18), wherein said hydraulic condenser means (11) preferably comprises an elastic chamber.

**7.** Device according to any one of the preceding claims, **characterised in that** the irrigation line (5) is fed by at least one source (13) of irrigation fluid, whereby said control means (12) opens said valve means (7; 18) when the pressure sensed by said sensor means (9; 20) is lower than the second pressure value, thus putting the chamber (6; 17) in communication with said end portion of the irrigation line (5), wherein said at least one source preferably comprises a "gravity" system using a hydrostatic pressure of the irrigation fluid contained within a further container (13) placed at a certain height.

**8.** Device according to any one of the preceding claims, **characterised in that** it further comprises a suction line (15).

**9.** Device according to any one of the preceding claims, **characterised in that** the handpiece (14) internally includes the irrigation line (5), said valve means (18), said control means (12), and said sensor means (9; 20), said end portion of the irrigation line (5) comprising an irrigation sleeve (19) coupled to the handpiece (14).

**10.** Device according to claim 9, when dependent on claim 6, **characterised in that** said hydraulic condenser means (11) is constituted by the irrigation sleeve (19).

**11.** Device according to claim 9 or 10, when dependent on claim 8, **characterised in that** the handpiece (14) has substantially cylindrical shape, and the suction line (15) is coaxial to the handpiece (14).

**12.** Device according to any one of claims 9 to 11, **characterised in that** the handpiece (14) ends with a tip (1) having circular symmetry provided with a distal diaphragm (3) provided with at least one helicoidal scoring (2) ending with an external taper generating a circular cutting profile.

**13.** Device according to claim 12, when dependent on claim 8, **characterised in that** it is capable to aspirate a cataract and **in that** the tip (1) is operated so as to perform a reciprocating rotary motion capable to generate, due to inertial effect, a rotation of a cataract with respect to the tip (1).

**14.** Device according to claim 13, **characterised in that** said reciprocating rotary motion is asymmetrical, having different values $a_1$ and $a_2$ of angular acceleration in two directions of angular rotation, wherein the tip (1) preferably performs a rotation by an angle $\beta$ in the two directions of rotation, whereby the time intervals $t_1$ and $t_2$ necessary for covering the angle $\beta$ in the two directions are tied to the angular accelerations $a_1$ and $a_2$ by the relationship

$$a_1/a_2 = t_2^2/t_1^2$$

**15.** Device according to claim 13 or 14, **characterised in that** the tip (1) is operated by an electric and/or pneumatically operated and/or piezoelectric motor, preferably integrated in the handpiece (14).

**Patentansprüche**

**1.** Vorrichtung für ophthalmische Chirurgie, insbesondere zur Kataraktentfernung, mit intraokularer Druckstabilisation (IOP-Stabilisation), aufweisend ein Handstück (14), intern umfassend eine Irrigationsleitung (5), in der ein Irrigationsfluid strömt, aufweisend einen Endabschnitt, in der Lage, ein Auge zu irrigieren, wobei das Handstück (14) zusätzlich eine erste Quelle an Irrigationsfluid umfasst, umfassend eine Kammer (6; 17), wobei das Irrigationsfluid auf einem ersten Druckwert gehalten wird, wobei die Kammer (6; 17) verbunden ist durch eine Ventileinrichtung (7; 18) mit dem Endabschnitt der Irrigationsleitung (5), wobei die Ventileinrichtung (7; 18) intern in dem Handstück umfasst ist und gesteuert wird durch eine Steuereinrichtung (12) auf Basis einer Druckdetektion, bereitgestellt durch eine Drucksensoreinrichtung (9; 20), verbunden mit dem Endabschnitt der Irrigationsleitung (5) und in der Lage, einen intraokularen Druck (IPO) zu erfassen, wobei die Steuereinrichtung (12) die Ventileinrichtung (7; 18) öffnet, wenn der Druck, erfasst durch die Sensoreinrichtung (9; 20), nicht höher ist als ein zweiter Druckwert, der nicht höher ist als der erste Druckwert, wobei somit die Kammer (6; 18) in Kommunikation mit dem Endabschnitt der Irrigationsleitung (5) gebracht wird.

2. Vorrichtung gemäß Anspruch 1, **dadurch gekennzeichnet, dass** der zweite Druckwert niedriger ist als der erste Druckwert.

3. Vorrichtung gemäß Anspruch 1 oder 2, **dadurch gekennzeichnet, dass** einer oder beide der ersten und zweiten Druckwerte einstellbar ist.

4. Vorrichtung gemäß einem der vorhergehenden Ansprüche, **dadurch gekennzeichnet, dass** sie ferner einen Generator (10) umfasst, in der Lage, das Irrigationsfluid in der Kammer (5) auf dem ersten Druckwert zu halten, wobei der Generator (10) vorzugsweise Folgendes umfasst:

   - ein "Gravitations"-System, verwendend einen hydrostatischen Druck des Irrigationsfluids, enthalten in einem Behälter, platziert auf einer bestimmten Höhe, und/oder
   - einen dynamischen oder volumetrischen hydraulischen Generator, vorzugsweise eine hydraulische Pumpe.

5. Vorrichtung gemäß einem der vorhergehenden Ansprüche, **dadurch gekennzeichnet, dass** die Ventileinrichtung (17; 18) ein Solenoidventil umfasst und dadurch, dass die Steuereinrichtung eine elektronische Einheit (12) zum Steuern des Solenoidventils umfasst.

6. Vorrichtung gemäß einem der vorhergehenden Ansprüche, **dadurch gekennzeichnet, dass** sie ferner eine hydraulische Kondensatoreinrichtung (11) umfasst, verbunden mit dem Endabschnitt der Irrigationsleitung (5) nach der Ventileinrichtung (7; 18), wobei die hydraulische Kondensatoreinrichtung (11) vorzugsweise eine elastische Kammer umfasst.

7. Vorrichtung gemäß einem der vorhergehenden Ansprüche, **dadurch gekennzeichnet, dass** die Irrigationsleitung (5) versorgt wird durch zumindest eine Quelle (13) an Irrigationsfluid, wobei die Steuereinrichtung (12) die Ventileinrichtung (7; 18) öffnet, wenn der Druck, erfasst durch die Sensoreinrichtung (9; 20), niedriger ist als der zweite Druckwert, wobei somit die Kammer (6; 17) in Kommunikation mit dem Endabschnitt der Irrigationsleitung (5) gebracht wird, wobei die zumindest eine Quelle vorzugsweise ein "Gravitations"-System umfasst, verwendend einen hydrostatischen Druck des Irrigationsfluids, enthalten in einem weiteren Behälter (13), platziert auf einer bestimmten Höhe.

8. Vorrichtung gemäß einem der vorhergehenden Ansprüche, **dadurch gekennzeichnet, dass** sie ferner eine Saugleitung umfasst.

9. Vorrichtung gemäß einem der vorhergehenden Ansprüche, **dadurch gekennzeichnet, dass** das Handstück (14) intern die Irrigationsleitung (5), die Ventileinrichtung (18), die Steuereinrichtung (12) und die Sensoreinrichtung (9; 20) umfasst, wobei der Endabschnitt der Irrigationsleitung (5) eine Irrigationshülse (19) umfasst, gekoppelt an das Handstück (14).

10. Vorrichtung gemäß Anspruch 9, wenn abhängig von Anspruch 6, **dadurch gekennzeichnet, dass** die hydraulische Kondensatoreinrichtung (11) gebildet wird durch die Irrigationshülse (19).

11. Vorrichtung gemäß Anspruch 9 oder 10, wenn abhängig von Anspruch 8, **dadurch gekennzeichnet, dass** das Handstück (14) eine im Wesentlichen zylindrische Form aufweist und die Saugleitung (15) koaxial ist zu dem Handstück (14).

12. Vorrichtung gemäß einem der Ansprüche 9 bis 11, **dadurch gekennzeichnet, dass** das Handstück (14) mit einer Spitze (1) endet, aufweisend eine Kreissymmetrie, versehen mit einem distalen Diaphragma (3), versehen mit zumindest einer schraubenförmigen Riefung (2), endend mit einer externen Verjüngung, erzeugend ein Kreisschnittprofil.

13. Vorrichtung gemäß Anspruch 12, wenn abhängig von Anspruch 8, **dadurch gekennzeichnet, dass** sie in der Lage ist, einen Katarakt zu aspirieren und dadurch, dass die Spitze (1) betätigt wird, um eine hin- und hergehende Drehbewegung auszuführen, in der Lage, aufgrund Trägheitswirkung eine Drehung eines Katarakts in Bezug auf die Spitze (1) zu erzeugen.

14. Vorrichtung gemäß Anspruch 13, **dadurch gekennzeichnet, dass** die hin- und hergehende Drehbewegung asymmetrisch ist, aufweisend unterschiedliche Werte $a_1$ und $a_2$ in Bezug auf eine Winkelbeschleunigung in zwei Richtungen bezüglich einer Winkeldrehung, wobei die Spitze (1) vorzugsweise eine Drehung um einen Winkel $\beta$ in die zwei Drehrichtungen ausführt, wobei die Zeitintervalle $t_1$ und $t_2$, erforderlich zum Abdecken des Winkels $\beta$ in die zwei Richtungen verknüpft sind mit den Winkelbeschleunigungen $a_1$ und $a_2$ durch die Beziehung

$$a_1/a_2 = t_2{}^2/t1{}^2.$$

**15.** Vorrichtung gemäß Anspruch 13 oder 14, **dadurch gekennzeichnet, dass** die Spitze (1) betätigt wird durch einen elektrischen und/oder pneumatisch betätigten und/oder piezoelektrischen Motor, vorzugsweise integriert in dem Handstück (14).

**Revendications**

**1.** Dispositif pour chirurgie ophtalmologique, en particulier pour ablation de la cataracte, avec stabilisation de la pression intra-oculaire (IOP), comprenant une pièce à main (14) comprenant intérieurement une ligne d'irrigation (5), à l'intérieur de laquelle s'écoule un fluide d'irrigation, comportant une partie d'extrémité susceptible d'irriguer un oeil, la pièce à main (14) comprenant de plus une première source de fluide d'irrigation comprenant une chambre (6 ; 17) dans laquelle le fluide d'irrigation est maintenu à une première valeur de pression, la chambre (6 ; 17) étant reliée par l'intermédiaire de moyens formant vanne (7 ; 18) à ladite partie d'extrémité de la ligne d'irrigation (5), lesdits moyens formant vanne (7 ; 18) étant inclus intérieurement dans la pièce à main et étant commandés par des moyens de commande (12) en fonction d'une détection de pression produite par des moyens formant capteur de pression (9 ; 20) reliés à ladite partie d'extrémité de la ligne d'irrigation (5) et susceptibles de détecter une pression intra-oculaire (IOP), grâce à quoi lesdits moyens de commande (12) ouvrent lesdits moyens formant vanne (7 ; 18) lorsque la pression détectée par lesdits moyens formant capteur (9 ; 20) n'est pas supérieure à une deuxième valeur de pression non supérieure à la première valeur de pression, de façon à mettre ainsi ladite chambre (6 ; 17) en communication avec ladite partie d'extrémité de la ligne d'irrigation (5).

**2.** Dispositif selon la revendication 1, **caractérisé en ce que** ladite deuxième valeur de pression est inférieure à ladite première valeur de pression.

**3.** Dispositif selon la revendication 1 ou 2, **caractérisé en ce que** l'une parmi lesdites première et deuxième valeurs de pression, ou les deux, sont réglables.

**4.** Dispositif selon l'une quelconque des revendications précédentes, **caractérisé en ce qu'**il comprend de plus un générateur (10) susceptible de maintenir ledit fluide d'irrigation à l'intérieur de la chambre (6) à ladite première valeur de pression, le générateur (10) comprenant de préférence :

  - un système "à gravité" utilisant une pression hydrostatique du fluide d'irrigation contenu à l'intérieur d'un récipient disposé à une certaine hauteur, et/ou
- un générateur hydraulique dynamique ou volumétrique, de préférence une pompe hydraulique.

**5.** Dispositif selon l'une quelconque des revendications précédentes, **caractérisé en ce que** lesdits moyens formant vanne (7 ; 18) comprennent une électrovanne, et **en ce que** lesdits moyens de commande comprennent une unité électronique (12) pour commander l'électrovanne.

**6.** Dispositif selon l'une quelconque des revendications précédentes, **caractérisé en ce qu'**il comprend de plus des moyens formant condensateur hydraulique (11) reliés à ladite partie d'extrémité de la ligne d'irrigation (5) après lesdits moyens formant vanne (7 ; 18), lesdits moyens formant condensateur hydraulique (11) comprenant de préférence une chambre élastique.

**7.** Dispositif selon l'une quelconque des revendications précédentes, **caractérisé en ce que** la ligne d'irrigation (5) est alimentée par au moins une source (13) de fluide d'irrigation, grâce à quoi lesdits moyens de commande (12) ouvrent lesdits moyens formant vanne (7 ; 18) lorsque la pression détectée par lesdits moyens formant capteur (9 ; 20) est inférieure à la deuxième valeur de pression, de façon à mettre ainsi la chambre (6 ; 17) en communication avec ladite partie d'extrémité de la ligne d'irrigation (5), ladite source au nombre d'au moins une comprenant de préférence un système "à gravité" utilisant une pression hydrostatique du fluide d'irrigation contenu à l'intérieur d'un récipient additionnel (13) disposé à une certaine hauteur.

**8.** Dispositif selon l'une quelconque des revendications précédentes, **caractérisé en ce qu'**il comprend de plus une ligne d'aspiration (15).

**9.** Dispositif selon l'une quelconque des revendications précédentes, **caractérisé en ce que** la pièce à main (14) comprend intérieurement la ligne d'irrigation (5), lesdits moyens formant vanne (18), lesdits moyens de commande (12) et lesdits moyens formant capteur (9 ; 20), ladite partie d'extrémité de la ligne d'irrigation (5) comprenant un manchon d'irrigation (19) couplé à la pièce à main (14).

**10.** Dispositif selon la revendication 9, lorsqu'elle dépend de la revendication 6, **caractérisé en ce que** lesdits moyens formant condensateur hydraulique (11) sont constitués par le manchon d'irrigation (19).

**11.** Dispositif selon la revendication 9 ou 10, lorsqu'elles dépendent de la revendication 8, **caractérisé en ce que** la pièce à main (14) a une forme sensiblement cylindrique, et **en ce que** la ligne d'aspiration (15) est coaxiale à la pièce à main (14).

12. Dispositif selon l'une quelconque des revendications 9 à 11, **caractérisé en ce que** la pièce à main (14) se termine par une pointe (1) ayant une symétrie circulaire, munie d'un diaphragme distal (3) comportant au moins une entaille hélicoïdale (2) se terminant en un effilement extérieur générant un profil de coupe circulaire.

13. Dispositif selon la revendication 12, lorsqu'elle dépend de la revendication 8, **caractérisé en ce qu'**il est susceptible d'aspirer une cataracte et **en ce que** la pointe (1) est actionnée de façon à effectuer un mouvement de va-et-vient rotatif susceptible de générer, grâce à un effet d'inertie, une rotation d'une cataracte par rapport à la pointe (1).

14. Dispositif selon la revendication 13, **caractérisé en ce que** ledit mouvement de va-et-vient rotatif est asymétrique, ayant différentes valeurs $a_1$ et $a_2$ d'accélération angulaire dans deux directions de rotation angulaire, la pointe (1) effectuant de préférence une rotation d'un angle $\beta$ dans les deux directions de rotation, grâce à quoi les intervalles de temps $t_1$ et $t_2$ nécessaires pour couvrir l'angle $\beta$ dans les deux directions sont liés aux accélérations angulaires $a_1$ et $a_2$ par la relation :

$$a_1/a_2 = t_2^2/t_1^2$$

15. Dispositif selon la revendication 13 ou 14, **caractérisé en ce que** la pointe (1) est actionnée par un moteur électrique et/ou actionné pneumatiquement et/ou piézoélectrique, de préférence intégré dans la pièce à main (14).

(b)

a2

a1

β

(a)

4

2

3

1

Fig. 1

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20010031951 A1, Pezzola
- US 20020099325 A1, Sutton
- US 6299591 B1, Banko
- US 5167620 A, Ureche
- US 4921477 A, Davis
- US 6042586 A, Kawano
- IT RM20060477 A, Optikon
- US 6491661 B1, Bouchny
- WO 9825557 A, Alcon
- US 2006084961 A
- WO 9812973 A
- DE 19718139

- CA 2113911
- WO 8906522 A
- US 5843071 A
- US 7041078 B
- US 2005251105 A, PEYMAN GHOLAM
- US 5695461 A, SCHAIBLE ERIC
- DE 4012882
- DE 1971167 A1
- US 5830176 A
- US 5722945 A
- EP 1212995 A, Optikon